# EUROPEAN PATENT APPLICATION

(11) **EP 4 740 956 A1**
(43) Date of publication of application: **13.05.2026**
(21) Application number: 25751687.2
(22) Date of filing: 08.02.2025
(51) Int. Cl.: A61K 38/47, A61K 47/68, A61K 9/08, A61P 35/00

(54) **PHARMACEUTICAL COMBINATION FOR SUBCUTANEOUS ADMINISTRATION AND USE THEREOF**

(30) Priority: 08.02.2024 WO PCT/CN2024/076995; 16.08.2024 WO PCT/CN2024/112586
(71) Applicant: Shanghai Bao Pharmaceuticals Co., Ltd., Shanghai 201908 (CN)
(72) Inventor: LIU, Yanjun, Shanghai 201908 (CN)
(74) Representative: Bayramoglu et al.
(86) International application number: PCT/CN2025/076396
(87) International publication number: WO 2025/168089

(57) **Abstract**

A pharmaceutical combination for subcutaneous administration and use thereof are provided. The pharmaceutical combination for subcutaneous administration comprises a hyaluronidase and an antibody-drug conjugate. The pharmaceutical combination can enable administration in a greater volume or at a higher dose, without worsened adverse effects.

## Description

### PRIORITY CLAIM

This application is a Continuation of International Patent Application No. PCT/CN2025/076396 filed on Feb. 8, 2025, which claims priority to International Patent Application No. PCT/CN2024/076995 entitled "SUBCUTANEOUS ANTIBODY-DRUG CONJUGATES", filed on Feb. 8, 2024, and International Patent Application No. PCT/CN2024/112586 entitled "PHARMACEUTICAL COMBINATIONS FOR SUBCUTANEOUS ADMINISTRATION AND USES THEREOF", filed on Aug. 16, 2024, which are incorporated herein by reference in their entireties.

### TECHNICAL FIELD

The present disclosure relates to the field of biopharmaceuticals, and in particular, to a pharmaceutical combination for subcutaneous administration and use thereof.

### BACKGROUND TECHNOLOGY

Existing antibody-drug conjugates are administered by intravenous (IV) injection. However, the IV administration faces a series of clinical challenges. For example, for patients with poor venous access, IV treatments may result in the loss of treatment opportunity. Injections need to be performed in an outpatient setting, which increases the risk of hospital-acquired infections. The need for long-term infusion and accompaniment may lead to the loss of laborers and additional medical insurance expenses such as hospitalization consumables and interventions for complications, increasing the complexity of drug formulation processes and the difficulty of quality and safety management. Moreover, the prolonged infusion time may also lead to a low patient turnover.

Very few cytotoxic agents can be administered subcutaneously for the treatment of cancers, as most known anti-cancer cytotoxic agents may cause local damage in the subcutaneous or dermal tissues after extravasation. In Chinese Patent No. CN110352201A, the local toxicity of ADCs is controlled by optimizing the dosage and time. However, subcutaneous formulations generally require higher doses relative to intravenous injections, which in turn require higher injection volumes and/or drug concentrations. Although a variety of FDA-approved antibodies have been developed as subcutaneous formulations, subcutaneous (sc) administration is difficult for antibody-drug conjugates (ADCs) since immune cells in the skin may respond to cytotoxic loads and mediate off-target effects, leading to toxic effects such as the local deposition of cytotoxic substances (Lucas AT, Price LSL, Schorzman AN, et al. Factors Affecting the Pharmacology of Antibody-Drug Conjugates. Antibodies (Basel, Switzerland). 2018 Feb; 7(1):E10. DOI: 10.3390/antib7010010.).

Also, as the volume of a conventional subcutaneous dose is generally less than 2 mL, formulations with a high concentration are required for ADCs. ADCs generally have higher hydrophobicity after the drug load is conjugated. Therefore, the development and production of pharmaceutical ADC formulations are also challenging, and the development of subcutaneous formulations with a high concentration is even more difficult. Due to the large variety of substances in conjugate products, the sample complexity makes the preparation of non-specific ADCs extremely difficult. In addition, the drugs and the linkers in ADCs also increase the complexity of preparing ADCs. During the conjugation of ADCs, aggregation-prone regions in the antibody backbone may be exposed, or the aggregation between antibodies may be caused by drug-mediated interactions, which greatly limits the ability to prepare high-stability and high-concentration ADC treatments.

Therefore, there is an urgent clinical need to develop ADC composition/combinations that enable safe and efficient subcutaneous administration.

### CONTENT OF THE INVENTION

In some aspects of the present disclosure, provided is a pharmaceutical combination comprising a hyaluronidase and an antibody-drug conjugate.

In some embodiments, the pharmaceutical combination disclosed herein is administered subcutaneously.

In some embodiments, the antibody-drug conjugate in the pharmaceutical combination disclosed herein comprises a bioactive molecule fragment.

In some embodiments, the bioactive molecule described herein is a bioactive molecule with moderate toxicity.

In some embodiments, the bioactive molecule described herein is a topoisomerase I inhibitor.

In some embodiments, the topoisomerase I inhibitor described herein is a camptothecin-based topoisomerase I inhibitor.

In some embodiments, the topoisomerase I inhibitor described herein is selected from camptothecin (CPT), hydroxycamptothecin (HCPT), 9-aminocamptothecin (9-AC), 7-ethyl-10-hydroxycamptothecin (SN-38), exatecan derivative (Dxd or DX-8951 derivative), irinotecan (CPT-11), topotecan, lurtotecan, belotecan, and exatecan.

In some embodiments, the topoisomerase I inhibitor described herein is selected from any one of the following:

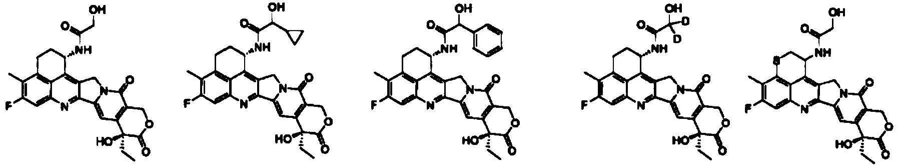

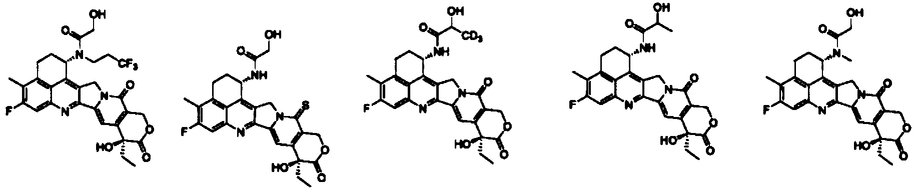

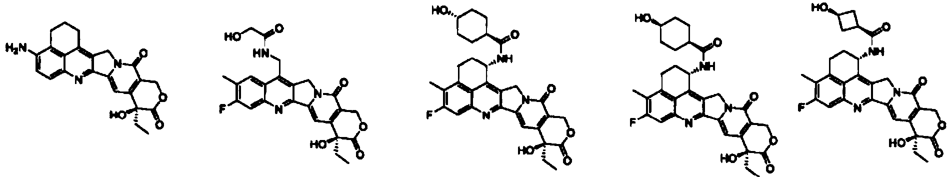

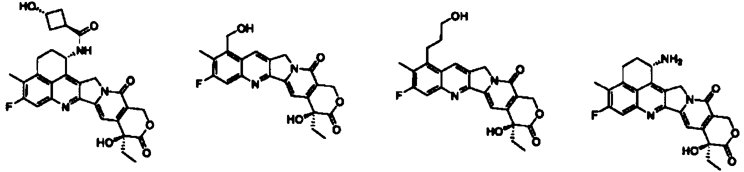

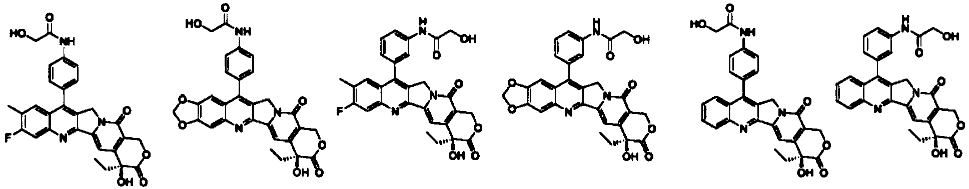

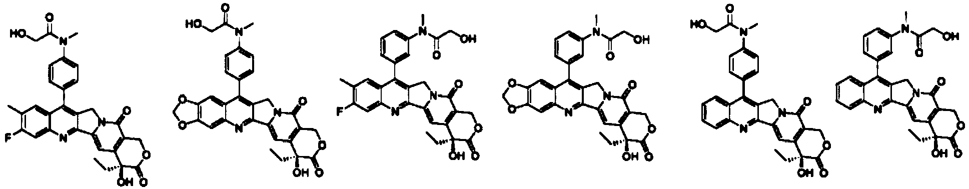

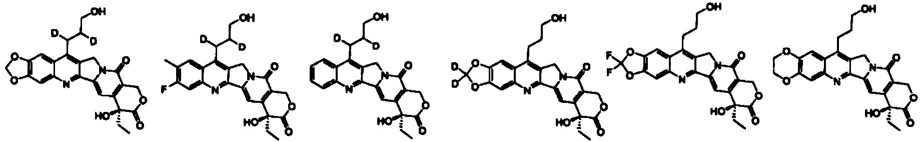

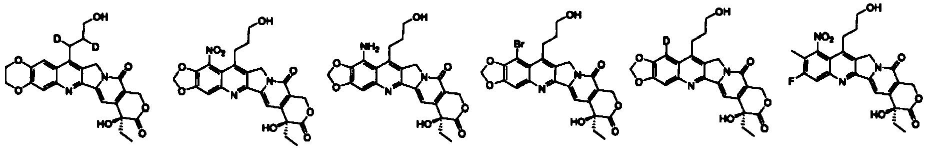

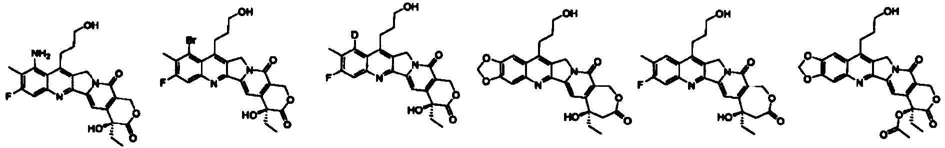

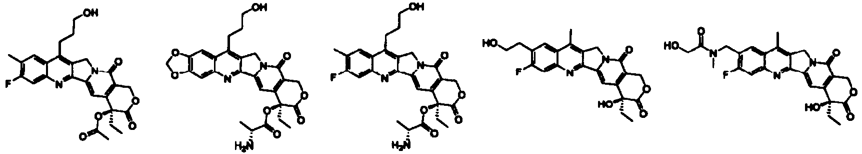

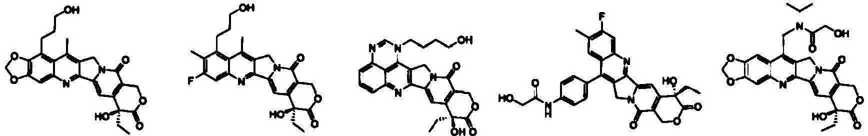

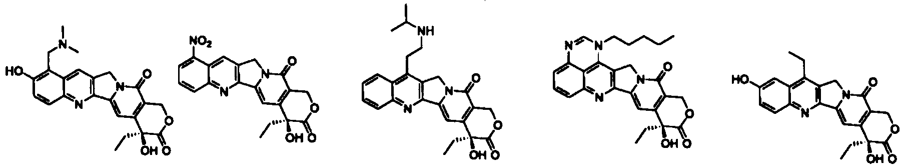

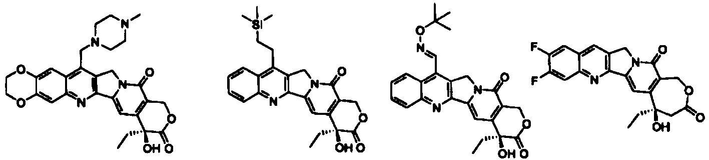

In some embodiments, the antibody-drug conjugate described herein is selected from: 9MW-2921, A-315, ACR-246, ADC-2154, AZD-9592, AZD-9829, BIO-201, BLB-01D1, BSI-04702, CUSP-06, DAN-311, DB-1303, DB-1311, GPCR-targeted Project 010, HDP-201, HLX-42, HLX-43, IBI-354, IM-1021, JSKN-033, M-9140, MABS-01, MBK-101, MBK-102, MBK-105, NV-104, OBI-902, OBI-904, OBI-905, PRO-1102, DS-6000, IMMU-132, SMP-190, TQB-2102, DS-8201, DS-1062, DS-7300, U3-1402, XB-033, YL-201, YL-202, ZW-191, ZW-220, and ZW-251.

In some embodiments, the hyaluronidase described herein is a mammalian hyaluronidase.

In some embodiments, the hyaluronidase described herein comprises the catalytic domain of hyaluronidase PH-20, HYAL1, HYAL2, HYAL3, HYAL4, or HYALPS1.

In some embodiments, the hyaluronidase described herein is selected from HuPH20, HYAL1, HYAL2, HYAL3, and HYAL4, or any variant thereof, or a hyaluronidase of any isotype thereof.

In some embodiments, the hyaluronidase described herein is rHuPH20, HYAL1, PH20 variant 1, or a fragment thereof.

In some embodiments, the pharmaceutical combination disclosed herein is selected from the group consisting of the following pharmaceutical combinations: rHuPH20 and DS-8201, rHuPH20 and IMMU-132, rHuPH20 and DS-1062, rHuPH20 and DS-7300, rHuPH20 and U3-1402, and PH20 variant 1 and DS-8201.

In some embodiments, the peak serum concentration (Cₘₐₓ (ng/mL)) of the pharmaceutical combination disclosed herein administered subcutaneously is about 5%-20%, preferably greater than about 5%, greater than about 8%, greater than about 10%, greater than about 12%, greater than about 14%, greater than about 16%, greater than about 18%, or greater than 19%, of the peak plasma concentration of the antibody-drug conjugate administered intravenously at the same dose.

In some embodiments, the half-life of the pharmaceutical combination disclosed herein administered subcutaneously is about 70%-99%, preferably greater than about 70%, greater than about 80%, greater than about 85%, greater than about 90%, or greater than about 95%, of the half-life of the antibody-drug conjugate administered intravenously at the same dose.

In some embodiments, the exposure (AUC_{0-∞} (h*ng/mL)) of the pharmaceutical composition disclosed herein administered subcutaneously is about 50%-70%, preferably greater than about 50%, greater than about 55%, greater than about 60%, greater than about 65%, or greater than about 67%, of the exposure of the antibody-drug conjugate administered intravenously at the same dose.

In some embodiments, in the pharmaceutical combination disclosed herein, the antibody-drug conjugate is administered at a concentration of about 2-100 mg/mL; the hyaluronidase is administered at a concentration of about 100-5000 IU/mL; optionally, the antibody-drug conjugate is administered at a concentration of about 3 mg/mL, 4 mg/mL, 5 mg/mL, 6 mg/mL, 8 mg/mL, 10 mg/mL, 12 mg/mL, 14 mg/mL, 16 mg/mL, 18 mg/mL, 20 mg/mL, 30 mg/mL, 40 mg/mL, 50 mg/mL, 60 mg/mL, 80 mg/mL, or 100 mg/mL; the hyaluronidase is administered at a concentration of about 100 IU/mL, 150 IU/mL, 200 IU/mL, 500 IU/mL, 1000 IU/mL, 2000 IU/mL, 3000 IU/mL, or 5000 IU/mL; optionally, the antibody-drug conjugate is administered at a concentration of about 20 mg/mL, and the hyaluronidase is administered at a concentration of about 500 IU/mL.

In some embodiments, in the pharmaceutical combination disclosed herein, the antibody-drug conjugate and the hyaluronidase are administered in a dose ratio of about 2-100 mg:100-5000 U; optionally, the antibody-drug conjugate and the hyaluronidase are administered in a dose ratio of about 3-60 mg:100-3000 U; optionally, the antibody-drug conjugate is administered at a dose of 3 mg/mL, 4 mg/mL, 5 mg/mL, 6 mg/mL, 10 mg/mL, 20 mg/mL, 30 mg/mL, 40 mg/mL, 50 mg/mL, or 60 mg/mL; optionally, the hyaluronidase is administered at a dose of 100 IU/mL, 150 IU/mL, 200 IU/mL, 500 IU/mL, 1000 IU/mL, 2000 IU/mL, or 3000 IU/mL; optionally, the ratio of the antibody-drug conjugate to the hyaluronidase is about 20 mg:500 IU.

In some embodiments, in the pharmaceutical combination disclosed herein, the antibody-drug conjugate is DS-8201.

In some embodiments, in the pharmaceutical combination disclosed herein, the pharmaceutical combination is: rHuPH20 and DS-8201; DS-8201 is administered at a concentration of about 20 mg/mL, and rHuPH20 is administered at a concentration of about 500 IU/mL.

In some embodiments, in the pharmaceutical combination disclosed herein, the pharmaceutical combination is: rHuPH20 and DS-8201; DS-8201 and rHuPH20 are administered in a dose ratio of about 20 mg:500 IU.

In some aspects of the present disclosure, provided is a pharmaceutical composition for subcutaneous administration, comprising: the pharmaceutical combination according to any one of the foregoing embodiments and a pharmaceutically acceptable excipient.

In some embodiments, in the pharmaceutical composition disclosed herein, the pharmaceutically acceptable excipient comprises one or more of a lyoprotectant, a stabilizer, a solubilizer, an osmotic pressure regulator, and a buffer.

In some embodiments, in the pharmaceutical composition disclosed herein, the pharmaceutically acceptable excipient comprises one or more of sucrose, L-histidine, L-histidine hydrochloride monohydrate, polysorbate, phosphate, methionine, trehalose, mannitol, and sodium chloride.

In some embodiments, in the pharmaceutical composition disclosed herein, the pharmaceutically acceptable excipient comprises one or more of sucrose, L-histidine, L-histidine hydrochloride monohydrate, and polysorbate 80.

In some embodiments, in the pharmaceutical composition disclosed herein, the pharmaceutically acceptable excipient further comprises one or more of disodium hydrogen phosphate, methionine, trehalose, mannitol, and polysorbate 20.

In some aspects of the present disclosure, provided is a method for treating a tumor, comprising: administering to a subject in need the pharmaceutical combination according to any one of the foregoing embodiments or the pharmaceutical composition according to any one of the foregoing embodiments.

In some embodiments, the tumor is lymphatic metastasis, breast ductal carcinoma, breast cancer, gastric cancer, ovarian cancer, or pancreatic cancer.

In some aspects of the present disclosure, provided is use of the pharmaceutical combination according to any one of the foregoing embodiments or the pharmaceutical composition according to any one of the foregoing embodiments in preparing a medicament for treating a tumor.

In some aspects of the present disclosure, provided is a method for improving the bioavailability of the drug conjugate according to any one of the foregoing embodiments, the method comprising: administering to a subject the hyaluronidase according to any one of the foregoing embodiments concurrently with or prior to the administration of the drug conjugate.

In some aspects of the present disclosure, provided is a method for alleviating local irritation caused by subcutaneous administration of the drug conjugate according to any one of the foregoing embodiments, the method comprising: administering to a subject the hyaluronidase according to any one of the foregoing embodiments simultaneously with or prior to the administration of the drug conjugate.

### DESCRIPTION OF THE DRAWINGS

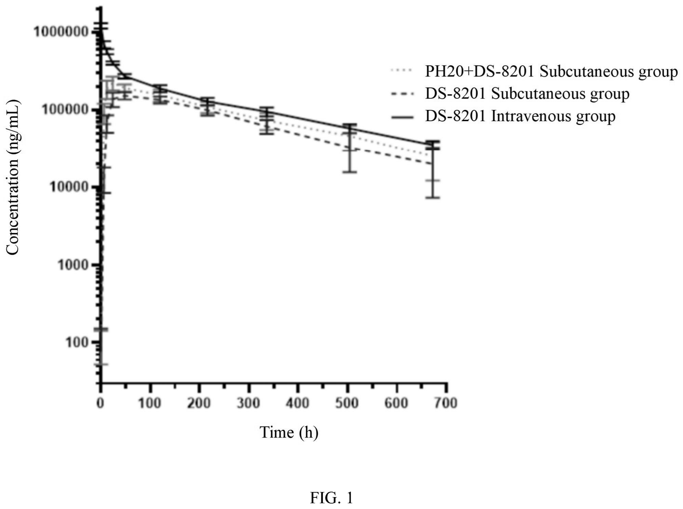

FIG. 1 illustrates the PK study results for ADC + PH20 in Example 1.

### SPECIFIC IMPLEMENTATIONS

### I. Description

Unless otherwise defined, all technical and scientific terms used herein have the same meaning as commonly understood by those of ordinary skill in the art to which the present disclosure pertains. The terminology used in the detailed description herein is for the purpose of describing particular embodiments only, rather than limiting.

All publications, patent applications, patents, GenBank or other accession numbers, and other references mentioned herein are incorporated by reference in their entireties.

Unless otherwise indicated in the context, it is specifically intended that the various features described herein can be used in any combination.

The following terms are used in the specification and the claims:
The term "a", "an", and/or "the" encompass singular forms and plural forms, unless otherwise clearly indicated in the context.

In addition, when referring to a measurable value (e.g., the value of the length of a polynucleotide or polypeptide sequence, dose, time, temperature, etc.), the term "about" or "approximately" as used herein is intended to encompass a variation of ±10%, ±5%, ±1%, ±0.5%, or even ±0.1% of the specified amount.

In addition, as used herein, "and/or" refers to any one of the listed items that is selected alternatively ("or"), as well as any and all possible combinations including two or more of the associated listed items.

Unless otherwise indicated herein, a numerical range used herein is a unified description for the combination of all individual values falling within the numerical range, and should be construed as each individual value being separately illustrated herein. For example, if a concentration range is stated as 1% to 50%, it denotes that numerical values such as 2% to 40%, 10% to 30%, or 1% to 3% are explicitly enumerated in the present disclosure. Such numerical ranges or individual values are merely specifically intended examples, and all individual values between the enumerated minimum and maximum, including the numerical values of the minimum and maximum, and all possible combinations including such numerical values should be considered as explicitly stated in this disclosure.

The term "immunoglobulin" refers to a class of globulins found in the blood (serum), tissue fluid, and other exocrine fluids of humans and animals, which possess antibody activity and a chemical structure similar to that of antibody molecules. In some embodiments, the immunoglobulin may be interpreted as an antibody, for example, an IgG, IgE, IgM, IgD, or IgA antibody.

As used herein, the term "antibody" refers to a polypeptide comprising at least an immunoglobulin light chain variable region or heavy chain variable region that specifically recognizes and binds to an antigen. The term "antibody" encompasses a variety of antibody structures, including but not limited to, monoclonal antibodies, polyclonal antibodies, single-chain or multi-chain antibodies, monospecific or multispecific antibodies (e.g., bispecific antibodies, trispecific antibodies, or multispecific antibodies against more targets), fully human or chimeric or humanized antibodies, and full-length antibodies and antibody fragments, so long as they exhibit the desired antigen-binding activity.

The term "antigen-binding fragment" (used interchangeably herein with "antibody fragment" and "antigen-binding moiety") of an antibody refers to an incomplete antibody molecule, comprising a portion of an intact antibody for binding to an antigen to which the intact antibody would bind. As will be appreciated by those skilled in the art, the antigen-binding moiety of an antibody generally comprises amino acid residues from a "complementarity determining region" or "CDR". The antigen-binding fragment may be prepared by recombinant DNA techniques, or by enzymatic or chemical cleavage of an intact antibody. The antigen-binding fragment includes, but is not limited to, Fab, scFab, Fab', F(ab')₂, Fab'-SH, Fv, single-chain Fv, diabody, triabody, tetrabody, minibody, and single-domain antibody VHH. For a more detailed description of the antibody fragment, see: Fundamental Immunology, W.E. Paul (ed.), Raven Press, N.Y. (1993); Shao Rongguang et al. (eds.), Antibody Drug Research and Application, People's Medical Publishing House (2013); Hollinger et al., PNAS USA 90:6444-6448 (1993); and Hudson et al., Nat. Med. 9:129-134 (2003).

In the present disclosure, the antibody includes murine, chimeric, humanized or fully human antibodies prepared using techniques well known to those skilled in the art. The recombinant antibody, e.g., chimeric and humanized monoclonal antibodies, comprises human-derived and non-human-derived portions. Recombinant antibodies can be acquired by standard recombinant DNA techniques and are useful antibodies. The chimeric antibody is a molecule in which different portions are derived from different animal species, such as a chimeric antibody having the variable regions derived from a murine monoclonal antibody, and the constant regions derived from a human immunoglobulin (see, e.g., U.S. Pat. No. 4,816,567 and U.S. Pat. No. 4,816,397, which are incorporated herein by reference in their entireties). The humanized antibody refers to an antibody molecule derived from a non-human species, having one or more complementarity determining regions (CDRs) derived from the non-human species and framework regions derived from a human immunoglobulin molecule (see U.S. Pat. No. 5,585,089, which is incorporated herein by reference in its entirety). These chimeric and humanized monoclonal antibodies can be prepared using recombinant DNA techniques well known in the art.

The "hyaluronidase" (HAase) is a class of glycosidases that can degrade hyaluronic acid and part of glycosaminoglycans and can be found throughout the animal kingdom, including wild-type hyaluronidases, mutant hyaluronidases, and their corresponding recombinant hyaluronidases (rHAases). Hyaluronidases vary in substrate specificity and mechanism of action.

There are three prominent hyaluronidases:
1. Mammalian hyaluronidase (EC3.2.1.35), which is an endo-β-N-acetylhexosaminidase with tetrasaccharides and hexoses as its major end products. They have hydrolytic and transglycosidase activity and are capable of degrading hyaluronic acid and chondroitin sulfate (CS), in particular C4-S and C6-S.
2. Bacterial hyaluronidase (EC4.2.99.1), which degrades hyaluronic acid and degrades chondroitin sulfate (CS) and dermatan sulfate (DS) to different extents. They are endo-β-N-acetylhexosaminidases that act by means of a β-elimination reaction and mainly produce disaccharide end products.
3. Hyaluronidase (EC3.2.1.36) from leeches, other parasites and crustaceans, which is an endo-β-glucuronidase producing tetrasaccharide and hexose end products by hydrolyzing β1-3 bonds.

The mammalian hyaluronidase can be further divided into two groups: neutral-active and acid-active enzymes. There are six hyaluronidase-like genes (HYALs) in the human genome: HYAL1, HYAL2, HYAL3, HYAL4, HYALP1, and PH20/SPAM1. HYALP1 is a pseudogene and HYAL3 has not exhibited enzymatic activity on any known substrates. HYAL4 is a chondroitinase with no activity on hyaluronic acid. HYAL1 is the prototype acid-active enzyme, and PH20 is the prototype neutral-active enzyme. Acid-active hyaluronidases, such as HYAL1 and HYAL2, lack catalytic activity at neutral pH. For example, HYAL1 is catalytically inactive above pH 4.5 *in vitro* (Frost et al., Anal Biochemistry, 1997). HYAL2 is an acid-active enzyme with very low specific activity *in vitro.*

Hyaluronidase-like enzymes can also be characterized as those locked on the plasma membrane via glycosylphosphatidylinositol anchors. However, there is a variation from species to species: For example, bovine PH20 is very loosely attached to the plasma membrane without being anchored by a phospholipase-sensitive anchor (Lalancette et al., Biol Reprod. 2001 Aug; 65(2):628-36). This unique feature of bovine hyaluronidase makes it possible to use soluble bovine testicular hyaluronidase as an extract for clinical applications. Other species of PH20 are lipid-anchored enzymes that are insoluble without the use of detergents or lipases. For example, human PH20 is anchored to the plasma membrane by a GPI anchor. Attempts have been made to prepare human PH20 DNA constructs that do not introduce a lipid anchor, but the resulting enzymes are catalytically inactive or insoluble (Arming et al., Eur J Biochem. 1997 Aug 1; 247(3):810-4). The natural hyaluronidase from cynomolgus sperm was found in both soluble and membrane-bound forms. The 64-kDa membrane-bound form is enzymatically active at pH 7.0, whereas the 54-kDa form is only active at pH 4.0 (Cherr et al., Dev Biol. 1996 Apr 10; 175(1):142-53). Thus, the soluble form of PH20 lacks enzymatic activity in neutral conditions. Examples of hyaluronidase include, but are not limited to, hyaluronidases disclosed in WO2020022791A1 and WO2013102144A2.

In one embodiment, the hyaluronidase is selected from HYAL1, HYAL2, HYAL3, HYAL4, HYALP1, and PH20/SPAM1. For example, the amino acid sequences of optional hyaluronidases are set forth in SEQ ID NOs: 1-3.

**Table 1. Amino acid sequences of hyaluronidases**

| | | |
|---|---|---|
| SEQ ID NO: 1 | Recombinant human hyaluronidase rHuPH20 | (SEQ ID NO. 1) |
| SEQ ID NO:2 | Hyaluronidase HYAL1 | FRGPLLPNRPFTTVWNANTQWCLERHGVDVDVSVFDVVANPG QTFRGPDMTIFYSSQLGTYPYYTPTGEPVFGGLPQNASLIAHLA RTFQDILAAIPAPDFSGLAVIDWEAWRPRWAFNWDTKDIYRQR SRALVQAQHPDWPAPQVEAVAQDQFQGAARAWMAGTLQLGR ALRPRGLWGFYGFPDCYNYDFLSPNYTGQCPSGIRAQNDQLG WLWGQSRALYPSIYMPAVLEGTGKSQMYVQHRVAEAFRVAVA AGDPNLPVLPYVQIFYDTTNHFLPLDELEHSLGESAAQGAAGV VLWVSWENTRTKESCQAIKEYMDTTLGPFILNVTSGALLCSQA LCSGHGRCVRRTSHPKALLLLNPASFSIQLTPGGGPLSLRGALS |
| | | LEDQAQMAVEFKCRCYPGWQAPWCERKSMW(SEQ ID NO. 2) |
| SEQ ID NO: 3 | Hyaluronidase PH20 variant 1 | FRAPPVIPNVPFLWAWNAPSEFCLGKFDEPLDMSLFSFIGSPRIN ATGQGVTIFYVDRLGYYPYIDSITGVTVNGGIPQKISLQDHLDK AKKDITFYMPVDNLGMAVIDWEEWRPTWARNWKPKDVYKN RSIELVQQQNVQLSLTEATEKAKQEFEKAGKDFLVETIKLGKLL RPNHLWGYYLFPDCYNHHYKKPGYNGSCFNVEIKRNDDLSWL WNESTALYPSIYLNTQQSPVAATLYVRNRVREAIRVSKIPDAKSP LPVFAYTRIVFTDQVLKFLSQDELVYTFGETVALGASGIVIWGS WENTRTKESCQAIKEYMDTTLNPYIINVTLAAKMCSQVLCQE QGVCIRKNWNSSDYLHLNPDNFAIQLEKGGKFTVRGKPTLEDL EQFSEKFYCSCYSTLSCKEKADVKDTDAVDVCIADGVCIDAF |

The antibody-drug conjugates (ADCs) are a class of targeted biological agents consisting of an antibody, a linker, and a cytotoxic agent, and are intended to conjugate a target-specific monoclonal antibody with a highly cytotoxic agent via a specific linker. ADCs shall generally comprise the following 3 components: an antibody with high specificity and affinity, a linker with high stability, and an efficient small-molecule cytotoxic drug. ADC products include: Kadcyla^{®} (ado-trastuzumab emtansine), Enhertu^{®} (famtrastuzumab deruxtecannxki), Aidixi^{®} (disitamab vedotin), Padcev^{®} (enfortumab vedotin-ejfv), Trodelvy^{®} (sacituzumab govitecan hziy), Akalux^{®} (cetuximab saratolacan sodium), Tivdak^{®} (tisotumab vedotin-tftv), Elahere^{®} (mirvetuximab soravtansine-gynx), Mylotarg^{®}, Adcetris^{®}, Besponsa^{®}, Lumoxiti^{®}, Polivy^{®}, Blenrep^{®}, Zynlonta^{®}; 9MW-2921 (Mabwell Shanghai Bioscience Co. Ltd), A-315 (KLUS Pharma Inc), ACR-246 (Hangzhou Adcoris Biopharmaceutical Co. Ltd), ADC-2154 (Hangzhou Adcoris Biopharmaceutical Co. Ltd), AZD-9592 (AstraZeneca Plc), AZD-9829 (AstraZeneca Plc), BIO-201 (BiOneCure Therapeutics Inc), BLB-01D1 (Bristol-Myers Squibb Co., Systimmune Inc), BSI-04702 (Biosion Inc OBI Pharma Inc), CUSP-06 (Multitude therapeutics Inc, OnCusp Therapeutics Inc), DAN-311 (Dantari Inc), DB-1303 (BioNTech SE Duality Biologics (Shanghai) Co. Ltd), DB-1311 (BioNTech SE, Duality Biologics (Shanghai) Co. Ltd), GPCR-targeted Project 010 (Protheragen Inc), HDP-201 (Heidelberg Pharma AG), HLX-42 (Shanghai Henlius, Biotech Inc), HLX-43 (Shanghai Henlius Biotech Inc), IBI-354 (Innovent Biologics Inc), IM-1021 (Immunome Inc), JSKN-033 (Alphamab Oncology), M-9140 (Merck KGaA), MABS-01 (Manhattan Biosolutions Inc), MBK-101 (Mablink Bioscience SAS), MBK-102 (Mablink Bioscience SAS), MBK-105 (Mablink Bioscience SAS), Monoclonal Antibody Conjugate to Inhibit TOP1 and CD155 for Unspecified Cancer (Tasrif Pharmaceutical Inc), Next-Generation ADCs (Inxmed (Nanjing) Co. Ltd), NV-104 (NanoValent Pharmaceuticals Inc (Inactive)), OBI-902 (OBI Pharma Inc), OBI-904 (OBI Pharma Inc), OBI-905 (OBI Pharma Inc), PRO-1102 (ProfoundBio Suzhou Co. Ltd), raludotatug deruxtecan (Daiichi Sankyo Co. Ltd, DS-6000), sacituzumab govitecan (Immunomedics, IMMU-132), SMP-190 (Xiling Lab Co. Ltd), TQB-2102 (Chia Tai Tianqing Pharmaceutical Group Co. Ltd), trastuzumab deruxtecan (Daiichi Sankyo Co. Ltd, AstraZeneca, DS-8201), datopotamab deruxtecan (Daiichi Sankyo Co. Ltd, AstraZeneca, DS-1062), Ifinatamab deruxtecan (Daiichi Sankyo Co. Ltd, DS-7300), Patritumab deruxtecan (Daiichi Sankyo Co. Ltd, Merck & Co., Inc., U3-1402), XB-033 (Exelixis Inc), YL-201 (Suzhou Medilink Therapeutics Ltd), YL-202 (Suzhou Medilink Therapeutics Ltd), ZW-191 (Zymeworks Inc), ZW-220 (Zymeworks Inc), and ZW-251 (Zymeworks Inc).

### Drug

As used herein, the "drug" loosely refers to any compound that has desirable bioactivities and has a reactive functional group such that the conjugates described herein can be prepared. Desirable bioactivities include diagnosing, curing, alleviating, treating, and preventing diseases in humans or other animals. Therefore, it is necessary to have essential reactive functional groups. The compounds to which the term "drug" relates include drugs identified in the official national pharmacopeia as well as, e.g., the official Homeopathic Pharmacopoeia of the United States, the official National Formulary, or any supplements thereof. Typical drugs are set forth in the Physicians' Desk Reference (PDR) and the Orange Book by the U.S. Food and Drug Administration (FDA). It will be appreciated that as new drugs are constantly discovered and developed, such drugs shall also be encompassed in the "drug" in the drug conjugate of the present disclosure.

Drugs those can be used to construct the ADC of the present disclosure include, but are not limited to: cytotoxic drugs and non-cytotoxic drugs.

The term "cytotoxic drug" is equivalent to "cytotoxic agent". The "payload" refers to a substance that inhibits cell expression activity, disrupts cell functionality, and/or induces cell destruction. The term includes radioactive isotopes, chemotherapeutic agents, and toxins, such as small-molecule toxins or enzymatically active toxins of bacterial, fungal, plant or animal origins, including fragments and/or variants thereof. Examples of the cytotoxic drugs include, but are not limited to, dolastatins (e.g., auristatin), camptothecin and derivatives thereof, calicheamicins, adriamycin, duocarmycin, hemiasterlin, IGN, PBD, amanitin, clezutoclax, diphtheria toxin, eribulin, irinotecan, ricin, saporin, azonafide, amberstatin 269, dmDNA31, clezutoclax, belotecan, vinblastine, DM21C, Nigrin, glycosides (e.g., cardiac glycosides), and the like.

The term "moderate toxicity" generally refers to toxicity with an IC₅₀ value of nanomolar scale (0.1-10 nM). Common agents with moderate toxicity include auristatin derivatives such as MMAE/MMAF, and camptothecin derivatives such as DXd and SN-38.

The non-cytotoxic drugs include: glucocorticoids, photosensitizers (e.g., IRDye^{®} 700DX), Bcl-xL inhibitors, NAMPT inhibitors, TLR7/8 agonists, STING agonists, radiolabeled elements (e.g., iodine, lutetium, yttrium, indium), and the like.

In some certain aspects, cytotoxic agents are further classified as tubulin inhibitors, DNA damaging agents, topoisomerase I inhibitors, and the like. Further, the tubulin inhibitors include dolastatins, such as MMAE and MMAF; maytansinoids, such as maytansine, DM1, and DM4; halichondrins, such as halichondrin B, eribulin, and eribulin mesylate. The DNA damaging agents include calicheamicins and pyrrolobenzodiazepines (PBDs). The topoisomerase 1 inhibitors include camptothecins.

**Table 2. Common cytotoxic drugs**

| Targets | Mechanism of action | Payload | Representative product |
|---|---|---|---|
| Tubulin | Tubulin inhibitor | Dolastatins: MMAE, MMAF | Adcetris^{®}, Polivy^{®}, Padcev^{®} |
| | | Maytansines: DM1, DM4 | Kadcyla^{®} |
| | | Tubulysins | EC1248 (clinical) |
| | | Halichondrins: eribulin | Farletuzumab (clinical) |
| DNA | DNA damaging agent | Calicheamicins | Besponsa^{®} |
| | | PBDs | Zynlonta^{®} |
| | | Duocarmycins: duocarmycin | SYD985, SYD1875, MGC018 |
| | Topoisomerase I inhibitors | Camptothecins: SN38, Dxd | Trodelvy^{®}, Enhertu^{®} |

Camptothecin-based topoisomerase I inhibitors: camptothecins are currently the most effective and the most promising class of alkaloid anti-tumor drugs. Camptothecin molecules generally comprise five fused rings A, B, C, D, and E, wherein rings A and B are quinoline rings, ring C is a pyrrole ring, ring D is a pyridone, and ring E is an α-hydroxylactone with an S chiral carbon.

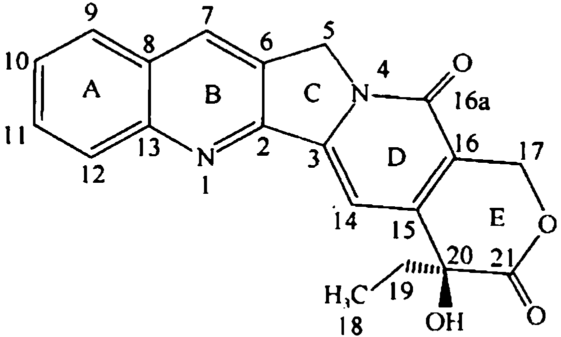

Common camptothecin chemicals include camptothecin (CPT), hydroxycamptothecin (HCPT), 9-aminocamptothecin (9-AC), 7-ethyl-10-hydroxycamptothecin (SN-38), exatecan derivative (Dxd or DX-8951 derivative), irinotecan (CPT-11), topotecan, lurtotecan, belotecan, and exatecan. Exemplary camptothecin-based topoisomerase I inhibitors also include camptothecin and derivatives thereof disclosed in CN117398474A, for example:

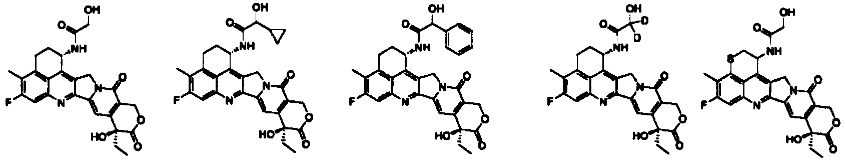

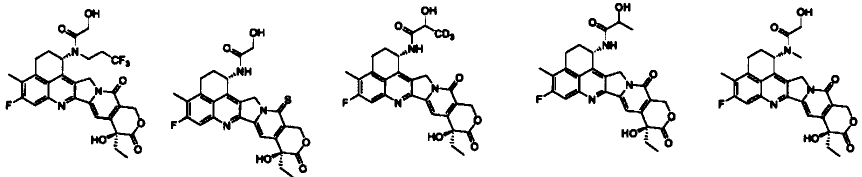

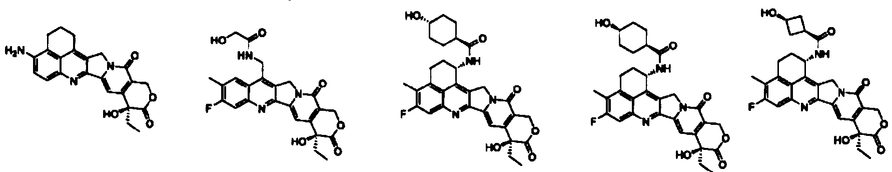

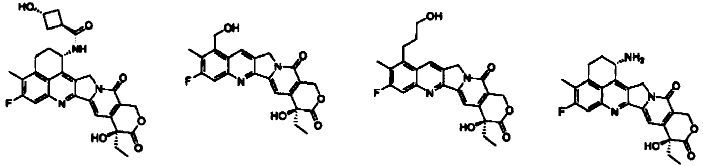

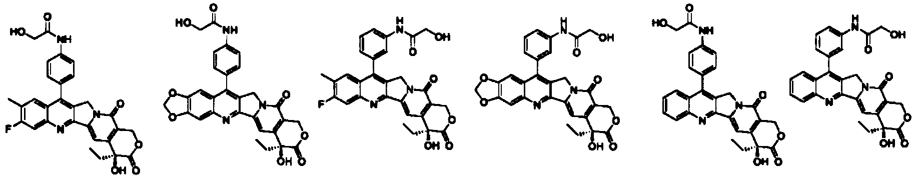

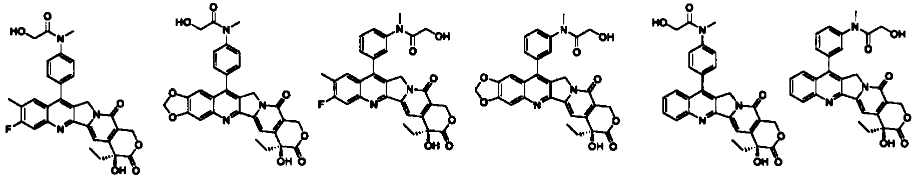

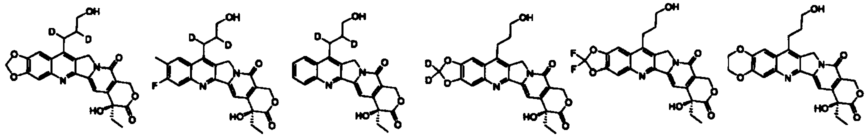

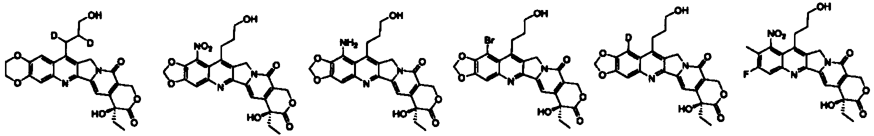

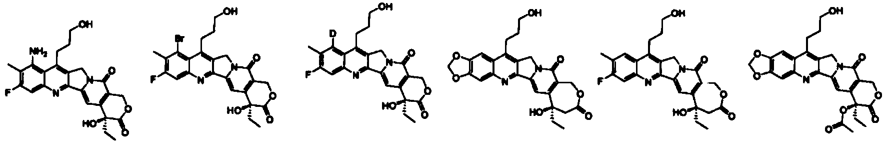

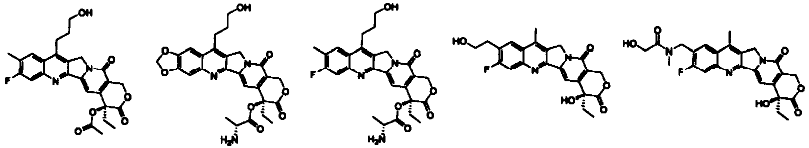

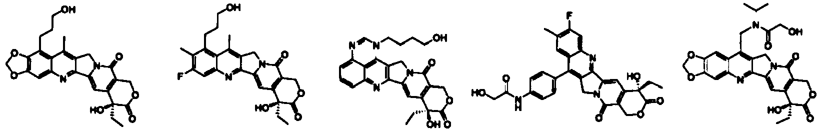

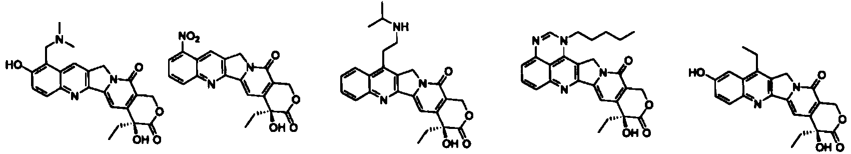

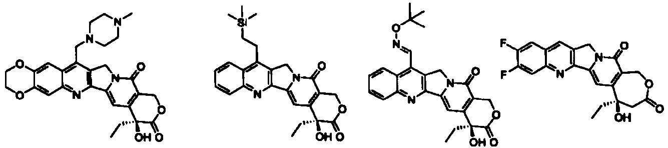

According to the intracellular drug release mechanism, the "linker" or "linker of the antibody-drug conjugate" described herein can be divided into two categories: non-cleavable linkers and cleavable linkers.

For an antibody-drug conjugate comprising a non-cleavable linker, the drug release mechanism is as follows: after the conjugate binds to an antigen and is endocytosed by a cell, the antibody is digested in lysosomes to release an active molecule consisting of the small molecule drug, the linker, and amino acid residues of the antibody. The resulting structural change of the drug molecule does not reduce its cytotoxicity, but the active molecule cannot infiltrate neighboring cells because it is charged (by amino acid residues). Thus, such active drugs will not kill adjacent tumor cells that do not express the targeted antigen (antigen-negative cells) (bystander effect).

The cleavable linker, as the name implies, can be cleaved in the target cell to release the active drug (the small molecule drug). The cleavable linkers can be divided into two major categories: chemically labile linkers and enzyme-labile linkers. The chemically labile linkers can be selectively cleaved due to the differences in properties of plasma and cytoplasm. Such properties include pH, glutathione concentration, etc. Linkers sensitive to pH are also known as acid-cleavable linkers. Such linkers are relatively stable in the neutral environment of the blood (pH 7.3-7.5), but will be hydrolyzed in weakly acidic endosomes (pH 5.0-6.5) and lysosomes (pH 4.5-5.0). Such linkers, e.g., hydrazones, carbonates, acetals, and ketals, are used in most of the first-generation antibody-drug conjugates. Due to the limited plasma stability of acid-cleavable linkers, the antibody-drug conjugates based on such linkers generally have a short half-life (2-3 days). Such a short half-life limits the application of pH-sensitive linkers in novel antibody-drug conjugates to some extent.

Linkers sensitive to glutathione are also known as disulfide linkers. The drug release is based on the difference between the high concentration of intracellular glutathione (in millimolar scale) and the low concentration of blood glutathione (in micromolar scale). Particularly, the low oxygen content in tumor cells leads to an increased activity of reductases and thus to higher glutathione concentrations. The disulfide bond has good thermodynamic stability, and thus has relatively good stability in plasma.

The enzyme-labile linkers, such as peptide linkers, enable better control of drug release. The peptide linkers are capable of being efficiently cleaved by intralysosomal proteases, such as cathepsin B or plasmin (with increased contents in some tumor tissues). Such a peptide linkage is believed to be very stable in the plasma circulation because proteases are generally not active due to the unfavorable pH outside the cell and serum protease inhibitors. In view of the high plasma stability and good intracellular cleavage selectivity and effectiveness, the enzyme-labile linkers are widely used as cleavable linkers in antibody-drug conjugates. Typical enzyme-labile linkers include Val-Cit (VC), Phe-Lys, and the like.

Self-immolative linkers are generally inserted between the cleavable linker and the active drug, or as a part of a cleavable linker. The mechanism of action of the self-immolative linkers is that when the cleavable linker is cleaved in suitable conditions, the self-immolative linkers can spontaneously undergo structural rearrangement to release the active drug linked thereto. Common self-immolative linkers include para-aminobenzyl (PAB), β-glucuronide, and the like.

### Pharmaceutical Composition and Route of Administration

The "pharmaceutical composition" described herein refers to a formulation or a combination of formulations comprising one, two, or more active ingredients, which allows the active ingredients contained therein to be present in an effective and bioactive form and does not contain additional ingredients having toxicity unacceptable to a subject to which the formulation is administered. The "pharmaceutical composition", when present in the form of a combination of separate formulations containing two or more different active ingredients, can be administered simultaneously, sequentially, separately, or intermittently, with the purpose of exerting the bioactivity of the multiple active ingredients for jointly treating the disease.

The pharmaceutical composition disclosed herein comprises a combination of at least the following components (1)-(3):
(1) a hyaluronidase;
(2) an ADC; and
(3) a formulation of one, two, or more active ingredients.

Another aspect of the disclosure relates to a method for treating a cancer in a subject in need, comprising: administering to the subject a therapeutically effective amount of a composition of the present disclosure, thereby treating the cancer.

Conventional methods, known to those of ordinary skill in the art of pharmaceuticals, can be used to administer the pharmaceutical composition to a subject, depending on the disease to be treated or the site of the disease. The composition may also be administered by other conventional routes, for example, oral, parenteral, by inhalational, topical, rectal, nasal, buccal or vaginal routes, or by implantation. The term "parenteral" as used herein includes subcutaneous, intradermal, intravenous, intramuscular, intraarticular, intraarterial, intrasynovial, intrasternal, intrathecal, intralesional, and intracranial injection or infusion techniques. In some certain cases, the route of administration may be subcutaneous. The subcutaneous administration comprises or does not comprise the administration of the hyaluronidase. The administration of the hyaluronidase may be performed before, after, or simultaneously with the ADC. As used herein, the term "simultaneously" refers to being sufficiently close in time to produce a synergic effect (i.e., "simultaneously" may mean occurring at the same time, or may mean two or more events occurring within a short period before or after one another).

In some embodiments, it may be desirable to administer the composition of the present disclosure to the subject more than once in order to provide a therapeutic effect or other beneficial effects. The composition of the present disclosure may be administered in, for example, 1, 2, 3, 4, or more doses.

In some embodiments, the composition disclosed herein further comprises at least one pharmaceutically acceptable carrier, excipient, and/or vehicle, such as solvents, buffering agents, solutions, dispersion media, coatings, antibacterial and antifungal agents, isotonic agents, and absorption delaying agents. In some embodiments, the pharmaceutically acceptable carrier, excipient, and/or vehicle may include saline, buffered saline, glucose, water, glycerol, sterile isotonic aqueous buffers, phosphate-buffered solutions, amino acid buffers, bicarbonate-buffered solutions, and combinations thereof. In some embodiments, the pharmaceutically acceptable carrier, excipient, and/or vehicle includes phosphate-buffered saline, sterile saline, lactose, sucrose, calcium phosphate, dextran, agar, pectin, peanut oil, sesame oil, medical-grade mannitol, lactose, starch, magnesium stearate, sodium saccharin, cellulose, magnesium carbonate, polyols (e.g., glycerol, propylene glycol, liquid polyethylene glycol, etc), or suitable mixtures thereof. In some embodiments, the composition disclosed herein further comprises a minor amount of an emulsifying or wetting agent or a pH buffering agent. The formulation of the composition disclosed herein may be prepared for storage, for example, in the form of a lyophilized powder, slurry, aqueous solution or suspension, by mixing with a physiologically acceptable carrier, excipient, or stabilizer.

In some embodiments, the composition is in a solid form, such as a lyophilized powder suitable for reconstitution, a liquid solution, a suspension, an emulsion, a tablet, a pill, a capsule, a sustained-release formulation, or a powder. In some embodiments, the composition may be formulated using a liposome, a nanocapsule, a microparticle, a microsphere, a lipid particle, a vesicle, a nanosphere, a nanoparticle, etc., for delivery purposes.

When treated with the antibody-drug conjugate of the present disclosure, the delivery may be performed by methods conventional in the art. For example, it may be introduced into cells using liposomes, hydrogels, cyclodextrins, biodegradable nanocapsules, or bioadhesive microspheres. Alternatively, the nucleic acid or vector may be delivered locally by direct injection or by using an infusion pump. Other methods include various transport and carrier systems using conjugates and biodegradable polymers.

Unless otherwise indicated, the term "polypeptide" as used herein includes a peptide and a protein.

The term "nucleotide" or "polynucleotide" means a single-stranded or double-stranded deoxyribonucleotide, deoxyribonucleoside, ribonucleoside or ribonucleotide, and polymers thereof. Unless specifically limited, the terms encompass nucleic acids containing known analogs of natural nucleotides, where the analogs have binding properties similar to reference nucleic acids and are metabolized in a manner similar to natural nucleotides. Unless otherwise limited specifically, the terms also mean oligonucleotide analogs, including PNAs (peptide nucleic acids) and DNA analogs used in antisense techniques (phosphorothioate, phosphoramidate, etc.). Unless otherwise specified, a particular nucleic acid sequence also implicitly encompasses its conservatively modified variants (including, but not limited to, degenerate codon substitutions) and complementary sequences as well as explicitly specified sequences. Specifically, degenerate codon substitution can be achieved by generating a sequence in which the 3rd position of one or more selected (or all) codons is substituted with a mixed base and/or a deoxyinosine residue (Batzer et al., Nucleic Acid Res., 19:5081 (1991); Ohtsuka et al., J. Biol. Chem., 260:2605-2608 (1985); Cassol et al., (1992); Rossolini et al., Mol Cell. Probes, 8:91-98 (1994)).

The terms "polypeptide" and "protein" are used interchangeably herein to mean polymers of amino acid residues. In other words, the description of a polypeptide is equally applicable to the description of a peptide and a protein, and vice versa. The terms apply to natural amino acid polymers, and amino acid polymers in which one or more amino acid residues are non-natural encoded amino acids. As used herein, the terms encompass amino acid chains of any length, including full-length proteins (i.e., antigens), in which amino acid residues are linked via covalent peptide bonds.

The term "host cell" means a cell comprising the nucleotide of the present disclosure, regardless of the method used for insertion to produce a recombinant host cell, such as direct uptake, transduction, pairing, or other methods known in the art. An exogenous polynucleotide can be present as a non-integrated vector such as a plasmid, or can be integrated into the host's genome. The host cell may be a prokaryotic cell or a eukaryotic cell.

The term "transformation" means a process by which a heterologous DNA sequence is introduced into a host cell or organism.

The term "expression" means the transcription and/or translation of an endogenous gene or a transgene in a cell.

The beneficial effects of the present disclosure are at least as follows: the subcutaneous ADC composition of the present disclosure allows the administration in a greater volume or at a higher dose, and after the subcutaneous ADC is administered in combination with a hyaluronidase, the ADC has an elevated peak plasma concentration, a reduced time to peak, a prolonged half-life, and a higher exposure than the ADC administered alone. Moreover, the subcutaneous ADC composition of the present disclosure has reduced adverse effects (reduced number of individuals with erythema and skin edema) after administration, achieving better therapeutic effects.

### II. Examples

The technical solutions of the present disclosure are further illustrated with reference to the following specific examples. It will be appreciated by those skilled in the art that the examples are only intended to help understand the present disclosure and should not be construed as specific limitations to the present disclosure. Experimental procedures without specified conditions in the following examples were performed in accordance with conventional procedures and conditions, or in accordance with product instructions.

### Example 1. Preparation of recombinant human hyaluronidase (rHuPH20) protein

The CHO cells were subjected to suspension culture in a serum-free medium and to controlled fed-batch culture in a serum-free feed medium, and then gradually expanded to a 30 L reactor scale through shake flask culture.

Since day 3 or 4, the amount of feed medium added to the bioreactor each day was 2% to 5% of the actual culture volume in the bioreactor. The culture temperature was controlled at 35-37 °C, and the pH was controlled at 7.0 by supplementing with 10% (w/v) Na₂CO₃ and CO₂; the reactor aeration volume was controlled at 0.015-0.15 vvm; the rotation speed was controlled at 80-150 rpm; the dissolved oxygen value was controlled at 20%-40%. During the cell culture, samples were taken every day, and the temperature, pH, glucose concentration, lactic acid concentration, osmolarity, and protein expression were monitored; when the CHO cell viability was less than 80% or the culture period reached 14-20 days, the culture was stopped and the recombinant human hyaluronidase (rHuPH20) was obtained.

The obtained recombinant human hyaluronidase supernatant was subjected to chromatography and filtration in sequence to give the purified protein. A recombinant human hyaluronidase stock solution with a SEC purity of greater than 95% and an RP purity of greater than 85% were obtained. The amino acid sequence of recombinant human hyaluronidase (rHuPH20) is set forth in SEQ ID NO: 1.

### Example 2. Subcutaneous injection of recombinant human hyaluronidase (rHuPH20) and DS-8201

The recombinant human hyaluronidase (rHuPH20) prepared in Example 1 was used to observe the effects of the subcutaneous injection of hyaluronidase liquid formulations on the subcutaneous pressure infusion of DS8201 solution in back of nude mice. The infusion time was recorded and the infusion rate was calculated.

36 female BALB/c nude mice were randomly divided into 3 groups according to the body weight: group 1, the negative control group (normal saline + DS-8201), group 2, the recombinant human hyaluronidase liquid formulation group (50 units/injection site first, followed by injection of DS8201), and group 3, the recombinant human hyaluronidase liquid formulation + DS-8201 mixed group, with 12 animals in each group. In group 3, a recombinant human hyaluronidase solution at 300,000 units/mL was added to the DS-8201 solution in a ratio of 1:60 before administration. The mixture was well mixed and administered. After anesthesia, 2 symmetrically arranged injection sites were marked on the back with a marker pen. The recombinant human hyaluronidase liquid formulation or the positive control sample solution was administered by subcutaneous injection on the right side of the back, and a corresponding volume of normal saline was administered by subcutaneous injection on the left side of the back, serving as an negative control. One end of a PE tube with an inner diameter of 0.76 mm was connected to a 1 mL syringe containing a DS-8201 solution of a certain concentration, and the other end was connected to a 0.55 mm needle. A certain volume of DS8201 solution was subcutaneously injected at the administration site at pressures of 20 cm, 30 cm, and 40 cm of water (6 animals in each group at each pressure). The infusion time t (min) was recorded, and the infusion rate v (µL/min) was calculated.

In this experimental conditions, the recombinant human hyaluronidase liquid formulation can significantly increase the rate of subcutaneous infusion of the high-concentration DS-8201 solution in nude mice.

### Example 3. PK study of ADC + rHuPH20

24 SD rats of about 250 g were randomly divided into 4 groups, with 6 rats in each group, half male and half female. The rats were given a single intravenous dose of DS-8201 via the tail vein or a single subcutaneous dose of DS-8201 or rHuPH20 + DS-8201 on the back. The whole blood was collected at 5 min, 6 h, 12 h, 24 h, 2 days, 5 days, 9 days, 14 days, 21 days, and 28 days post-dose, left to stand for 2 h, and centrifuged to collect the serum. The serum samples were tested after collection. The specific grouping and study design are shown in Table 3 below.
The subcutaneous treatment group exhibited a lower exposure (Cₘₐₓ and AUC) than that of the group without rHuPH20. The results are shown in Table 4 and FIG. 1.

**Table 3. Grouping and design of PK study of ADC + rHuPH20**

| Group | Route of administration | DS-8201 (mg/kg) | Administration concentration (mg/mL) | Administration volume (mL/kg) | rHuPH20 (mL) | rHuPH20 concentration (IU/mL) | Number of doses | Number of animals |
|---|---|---|---|---|---|---|---|---|
| rHuPH20 + DS-8201 subcutaneous group | SC | 60 | 3 | 20 | 1 | 150 | 1 | 6 |
| DS-8201 subcutaneous group | SC | 60 | 3 | 20 | / | / | 1 | 6 |
| DS-8201 intravenous group | IV | 60 | 3 | 20 | / | / | 1 | 6 |

**Table 4. Results for PK study of ADC + rHuPH20**

| Group | Cₘₐₓ (ng/mL) | Tₘₐₓ (h) | T_{1/2} (h) | AUC₀₋ₜ (h*ng/mL) | AUC_{0-∞} (h*ng/mL) | F (%) |
|---|---|---|---|---|---|---|
| rHuPH20 + DS-8201 subcutaneous group | 222167.2 | 24 | 215.3 | 60473922.4 | 68286505.0 | 68.2 |
| DS-8201 subcutaneous | 151942.0 | 48 | 196.1 | 48411568.8 | 54032273.4 | 54.6 |
| group | | | | | | |
| DS-8201 intravenous group | 1198231.0 | / | 235.6 | 88693576.0 | 100650393.2 | / |

As can be seen from Table 4, after the subcutaneous administration of the ADC in combination with hyaluronidase, compared with the subcutaneous administration of the ADC alone, the peak plasma concentration was increased by 46.21%, the time to peak was reduced by 50% (24 h), the half-life was prolonged by 9.7% (19.2 h), and the exposure was increased by 26.4%. Compared with the intravenous administration of the ADC alone, the peak plasma concentration was about 18.5%, the half-life was about 91.4%, and the exposure was about 67.8%.

### Example 4. Toxicity Study

24 SD rats of about 250 g were randomly divided into 4 groups, with 6 rats in each group, half male and half female. The rats were given intravenous doses of DS-8201 via the tail vein or subcutaneous doses of the control group, DS-8201, or rHuPH20 + DS-8201 on the back. The treatments were given once every 2 weeks for 2 doses (day 1 and day 15). During the study, the state of the mice and the administration sites were monitored. The specific grouping and study design are shown in Table 5.

On D18 (72 h after the last dose), the administration sites (skin, subcutaneous tissue, and muscle tissue) of the animals in groups 3 and 4 and the blank control area (skin, subcutaneous tissue and muscle tissue) of the animals in group 3 were collected, subjected to conventional histological treatments such as fixation, paraffin embedding, sectioning and HE staining, and subjected to histopathological examination to evaluate the local irritation to the skin of rats. Bilateral sampling was conducted in both groups and the sites were distinguished between the left and right.

**Table 5. Grouping and design for toxicity study**

| Group | Route of administration | DS-8 201 (mg/kg) | Administration concentration (mg/mL) | Administration volume (mL/kg) | PH20 (mL) | Concentration (IU/mL) |
|---|---|---|---|---|---|---|
| Control group | SC | / | / | / | 1 | 150 |
| rHuPH20 + DS-8201 subcutaneous group | SC | 240 | 20 | 12 | 2 (1 mL for each of left and right) | 150 |
| DS-8201 subcutaneous group | SC | 240 | 20 | 12 | / | / |
| DS-8201 intravenous group | IV | 240 | 20 | 12 | / | / |

The test results are shown in Table 6, indicating that there were different local symptoms after the administration.

**Table 6. Results of toxicity study**

| Group | Symptoms | Incidence of symptoms | | | | | | | | | |
|---|---|---|---|---|---|---|---|---|---|---|---|
| | | D1~D4 | D5 | D8 | D9 | D10 | D11 | D12 | D14 | D16 | D18 |
| rHuPH20 + DS-8201 subcutaneous group | Erythema | 0/6 | 0/6 | 3/6 | 3/6 | 3/6 | 3/6 | 3/6 | 3/6 | 3/6 | 3/6 |
| | Edema | 0/6 | 0/6 | 0/6 | 0/6 | 0/6 | 0/6 | 0/6 | 0/6 | 0/6 | 1/6 |
| DS-8201 subcutaneous group | Erythema | 0/6 | 3/6 | 4/6 | 4/6 | 4/6 | 5/6 | 5/6 | 5/6 | 5/6 | 6/6 |
| | Edema | 0/6 | 0/6 | 0/6 | 0/6 | 0/6 | 0/6 | 0/6 | 0/6 | 0/6 | 1/6 |

The observation after the administration suggests that animals in the rHuPH20 + DS-8201 subcutaneous group exhibited a lower rate of erythema as compared with the DS-8201 subcutaneous group (50% vs. 100%), demonstrating that the administration of rHuPH20 reduced the toxicity and adverse effects. Therefore, the results indicate that rHuPH20 has advantages for reducing local irritation.

### Example 5. In vivo study

5.1 An animal model was established using MDA-MB-175 cells to conduct the in vivo ADC study.

MDA-MB-175 cells were cultured in a culture medium, and cells in the logarithmic growth phase were collected. On the day of grafting, the cells were grafted on the right side of the third milk pad of the mice in 0.2 mL of PBS with Matrigel (1:1) at 2 × 10⁷ cells/mouse according to the modeling design. A 0.36 mg estrogen tablet was implanted the day before grafting. For each model, the administration was started when the mean tumor volume of the tumor-bearing mice reached 250 mm³.

**Table 7. Grouping and design**

| Group | Treatment group | Dose (mg/kg) | Volume (µL/g) | Route of administration | Treatment cycle |
|---|---|---|---|---|---|
| 1 | PBS | - | 10 | i.v. | |
| 2 | T-DM1 | High dose, low dose | Different volumes | i.v. | |
| 3 | T-DM1 | High dose, low dose | Different volumes | sc | |
| 4 | DS8201 | High dose, low dose | Different volumes | i.v. | |
| 5 | DS8201 | High dose, low dose | Different volumes | sc | 2 doses/week |
| 6 | DS8201+rHuPH20 | High dose, low dose | Different volumes | i.v. | |
| 7 | DS8201+rHuPH20 | High dose, low dose | Different volumes | sc | |
| 8 | T-DM1+rHuPH20 | High dose, low dose | Different volumes | i.v. | |
| 9 | T-DM1+rHuPH20 | High dose, low dose | Different volumes | sc | |

### 5.2 An animal model was established using NCI-N87 cells to conduct the in vivo ADC study.

NCI-N87 cells were cultured in a culture medium, and cells in the logarithmic growth phase were collected. On the day of grafting, the cells were grafted on the right upper back of the mice in 0.1 mL of PBS with Matrigel (1:1) at 1 × 10⁷ cells/mouse according to the modeling design. For each model, the administration was started when the mean tumor volume of the tumor-bearing mice reached about 200 mm³.

**Table 8. Grouping and design**

| Group | Treatment group | Dose (mg/kg) | Volume (µL/g) | Route of administration | Treatment cycle |
|---|---|---|---|---|---|
| 1 | PBS | - | 10 | i.v. | |
| 2 | T-DM1 | High dose, low dose | Different volumes | i.v. | 2 doses/week |
| 3 | T-DM1 | High dose, low dose | Different volumes | sc | |
| 4 | DS8201 | High dose, low dose | Different volumes | i.v. | |
| 5 | DS8201 | High dose, low dose | Different volumes | sc | |
| 6 | DS8201+rHuPH20 | High dose, low dose | Different volumes | i.v. | |
| 7 | DS8201+rHuPH20 | High dose, low dose | Different volumes | sc | |
| 8 | T-DM1+rHuPH20 | High dose, low dose | Different volumes | i.v. | |
| 9 | T-DM1+rHuPH20 | High dose, low dose | Different volumes | sc | |

### 5.3 An animal model was established using SK-OV-3 cells to conduct the in vivo ADC study.

SK-OV-3 cells were cultured in a culture medium, and cells in the logarithmic growth phase were collected. On the day of grafting, the cells were grafted on the right upper back of the mice in 0.1 mL of PBS with Matrigel (1:1) at 1 × 10⁷ cells/mouse according to the modeling design. For each model, the administration was started when the mean tumor volume of the tumor-bearing mice reached about 200-250 mm³.

**Table 9. Grouping and design**

| Group | Treatment group | Dose (mg/kg) | Volume (µL/g) | Route of administration | Treatment cycle |
|---|---|---|---|---|---|
| 1 | PBS | - | 10 | i.v. | |
| 2 | T-DM1 | High dose, low dose | Different volumes | i.v. | |
| 3 | T-DM1 | High dose, low dose | Different volumes | sc | 2 doses/week |
| 4 | DS8201 | High dose, low dose | Different volumes | i.v. | |
| 5 | DS8201 | High dose, low dose | Different volumes | sc | |
| 6 | DS8201+rHuPH20 | High dose, low dose | Different volumes | i.v. | |
| 7 | DS8201+rHuPH20 | High dose, low dose | Different volumes | sc | |
| 8 | T-DM1+rHuPH20 | High dose, low dose | Different volumes | i.v. | |
| 9 | T-DM1+rHuPH20 | High dose, low dose | Different volumes | sc | |

### 5.4 An animal model was established using JIMT-1 cells to conduct the in vivo ADC study.

JIMT-1 cells were cultured in a culture medium, and cells in the logarithmic growth phase were collected. On the day of grafting, the cells were grafted on the right upper back of the mice in 0.1 mL of PBS at 5 × 10⁶ cells/mouse according to the modeling design. For each model, the administration was started when the mean tumor volume of the tumor-bearing mice reached about 200-250 mm³.

**Table 10. Grouping and design**

| Group | Treatment group | Dose (mg/kg) | Volume (µL/g) | Route of administration | Treatment cycle |
|---|---|---|---|---|---|
| 1 | PBS | - | 10 | i.v. | |
| 2 | T-DM1 | High dose, low dose | Different volumes | i.v. | |
| 3 | T-DM1 | High dose, low dose | Different volumes | sc | |
| 4 | DS8201 | High dose, low dose | Different volumes | i.v. | |
| 5 | DS8201 | High dose, low dose | Different volumes | sc | 2 doses/week |
| 6 | DS8201+rHuPH20 | High dose, low dose | Different volumes | i.v. | |
| 7 | DS8201+rHuPH20 | High dose, low dose | Different volumes | sc | |
| 8 | T-DM1+rHuPH20 | High dose, low dose | Different volumes | i.v. | |
| 9 | T-DM1+rHuPH20 | High dose, low dose | Different volumes | sc | |

### 5.5 An animal model was established using Capan-1 cells to conduct the in vivo ADC study.

Capan-1 cells were cultured in a culture medium, and cells in the logarithmic growth phase were collected. On the day of grafting, the cells were grafted on the right upper back of the mice in 0.1 mL of PBS with Matrigel (1:1) at 5 × 10⁶ cells/mouse according to the modeling design. For each model, the administration was started when the mean tumor volume of the tumor-bearing mice reached about 170 mm³.

**Table 11. Grouping and design**

| Group | Treatment group | Dose (mg/kg) | Volume (µL/g) | Route of administration | Treatment cycle |
|---|---|---|---|---|---|
| 1 | PBS | - | 10 | i.v. | |
| 2 | T-DM1 | High dose, low dose | Different volumes | i.v. | |
| 3 | T-DM1 | High dose, low dose | Different volumes | sc | |
| 4 | DS8201 | High dose, low dose | Different volumes | i.v. | |
| 5 | DS8201 | High dose, low dose | Different volumes | sc | 2 doses/week |
| 6 | DS8201+rHuPH20 | High dose, low dose | Different volumes | i.v. | |
| 7 | DS8201+rHuPH20 | High dose, low dose | Different volumes | sc | |
| 8 | T-DM1+rHuPH20 | High dose, low dose | Different volumes | i.v. | |
| 9 | T-DM1+rHuPH20 | High dose, low dose | Different volumes | sc | |

### Example 6. In vivo efficacy study for lymphatic metastasis

6.1 A breast cancer lymphatic metastasis model was established in NOD/SCID mice using an MDA-MB-231-Luc human breast cancer cell suspension.

Female NOD/SCID mice aged 8 weeks were anesthetized by inhalation (3% sevoflurane, 100% oxygen), before 3 × 10⁶ MDA-MB-231-Luc cells suspended in 100 µL of Matrigel (20%) were grafted subcutaneously into the left second mammary fat pad of the NOD/SCID female mice, so as to induce in situ MDA-MB-231 tumors. On D28 from tumor grafting, the bioluminescence images of MDA-MB-231-Luc cells were acquired by the IVIS^{®} technique to track tumor growth and ALN (axillary lymph node) metastasis. The administration was started when lymphatic metastases were detected, and different doses of PBS, DS8201, T-DM1, DS8201 + rHuPH20, and T-DM1 + rHuPH20 were given to the mice by intravenous or subcutaneous injection at the anterior footpad. The mice were then euthanized, and the ALNs were taken for FACS or H&E staining.

**Table 12. Grouping and design**

| Group | Treatment group | Dose (mg/kg) | Volume (µL/g) | Route of administration | Treatment cycle |
|---|---|---|---|---|---|
| 1 | PBS | - | Different volumes | iv | |
| 2 | PBS+rHuPH20 | - | Different volumes | sc | |
| 3 | DS8201 | High dose, low dose | Different volumes | iv | |
| 4 | DS8201 | High dose, low dose | Different volumes | sc | |
| 5 | DS8201+rHuPH20 | High dose, low dose | Different volumes | iv | 2 doses/week |
| 6 | DS8201+rHuPH20 | High dose, low dose | Different volumes | sc | |
| 7 | T-DM1 | High dose, low dose | Different volumes | iv | |
| 8 | T-DM1 | High dose, low dose | Different volumes | sc | |
| 9 | T-DM1+rHuPH20 | High dose, low dose | Different volumes | iv | |
| 10 | T-DM1+rHuPH20 | High dose, low dose | Different volumes | sc | |

6.2 In this study, a breast cancer lymphatic metastasis model was established in NOD/SCID mice using an MCF-7-Luc human breast cancer cell suspension.

Female NOD/SCID mice aged 8 weeks were anesthetized by inhalation (3% sevoflurane, 100% oxygen), before 3 × 10⁶ MCF-7-Luc cells suspended in 100 µL of Matrigel (20%) were grafted subcutaneously into the left second mammary fat pad of the NOD/SCID female mice, so as to induce in situ MCF-7 tumors. On D28 from tumor grafting, the bioluminescence images of MCF-7-Luc cells were acquired by the IVIS^{®} technique to track tumor growth and ALN (axillary lymph node) metastasis. The administration was started when lymphatic metastases were detected, and different doses of PBS, DS8201, T-DM1, DS8201 + rHuPH20, and T-DM1 + rHuPH20 were given to the mice by intravenous or subcutaneous injection at the anterior footpad. The mice were then euthanized, and the ALNs were taken for FACS or H&E staining.

**Table 13. Grouping and design**

| Group | Treatment group | Dose (mg/kg) | Volume (µL/g) | Route of administration | Treatment cycle |
|---|---|---|---|---|---|
| 1 | PBS | - | Different volumes | iv | |
| 2 | PBS+rHuPH20 | - | Different volumes | sc | |
| 3 | DS8201 | High dose, low dose | Different volumes | iv | |
| 4 | DS8201 | High dose, low dose | Different volumes | sc | |
| 5 | DS8201+rHuPH20 | High dose, low dose | Different volumes | iv | 2 doses/week |
| 6 | DS8201+rHuPH20 | High dose, low dose | Different volumes | sc | |
| 7 | T-DM1 | High dose, low dose | Different volumes | iv | |
| 8 | T-DM1 | High dose, low dose | Different volumes | sc | |
| 9 | T-DM1+rHuPH20 | High dose, low dose | Different volumes | iv | |
| 10 | T-DM1+rHuPH20 | High dose, low dose | Different volumes | sc | |

In conclusion, the examples of the present disclosure demonstrate that the subcutaneous ADC composition of the present disclosure allows the administration in a greater volume or at a higher dose, and after the subcutaneous ADC is administered in combination with a hyaluronidase, the ADC has an elevated peak plasma concentration, a reduced time to peak, a prolonged half-life, and a higher exposure than the ADC administered alone. Moreover, the subcutaneous ADC composition of the present disclosure has reduced adverse effects (reduced number of individuals with erythema and skin edema) after administration, achieving better therapeutic effects.

## Claims

1. A pharmaceutical combination, comprising a hyaluronidase and an antibody-drug conjugate.

2. The pharmaceutical combination according to claim 1, wherein the pharmaceutical combination is administered subcutaneously.

3. The pharmaceutical combination according to claim 1 or 2, wherein the antibody-drug conjugate comprises a fragment of a bioactive molecule.

4. The pharmaceutical combination according to claim 3, wherein the bioactive molecule is a bioactive molecule with moderate toxicity.

5. The pharmaceutical combination according to claim 3 or 4, wherein the bioactive molecule is a topoisomerase I inhibitor.

6. The pharmaceutical combination according to claim 5, wherein the topoisomerase I inhibitor is a camptothecin-based topoisomerase I inhibitor.

7. The pharmaceutical combination according to claim 6, wherein the topoisomerase I inhibitor is selected from camptothecin (CPT), hydroxycamptothecin (HCPT), 9-aminocamptothecin (9-AC), 7-ethyl-10-hydroxycamptothecin (SN-38), exatecan derivative (Dxd or DX-8951 derivative), irinotecan (CPT-11), topotecan, lurtotecan, belotecan, or exatecan.

8. The pharmaceutical combination according to claim 6 or 7, wherein the topoisomerase I inhibitor is selected from any one of the following:

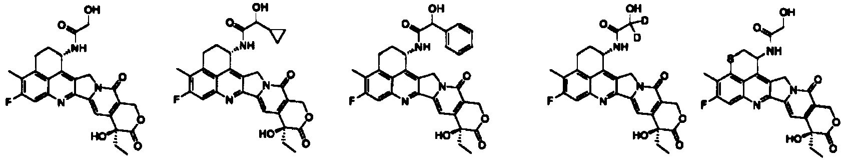

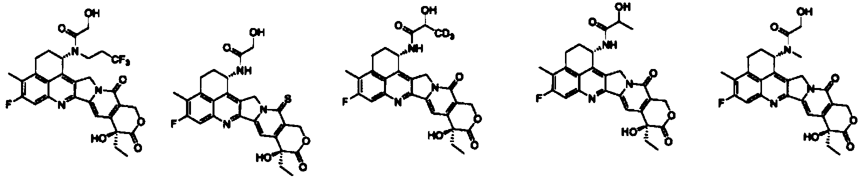

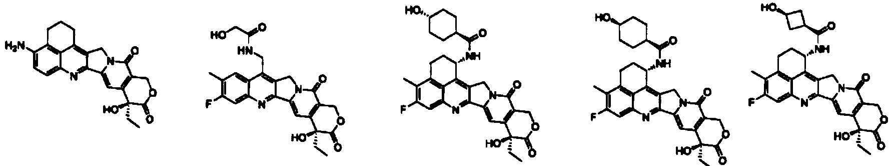

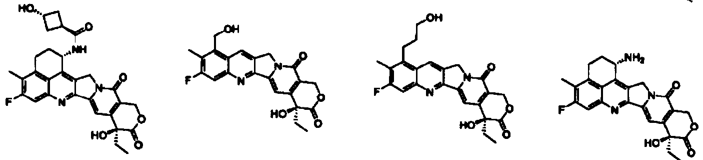

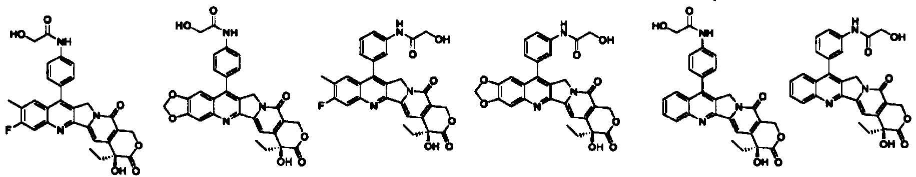

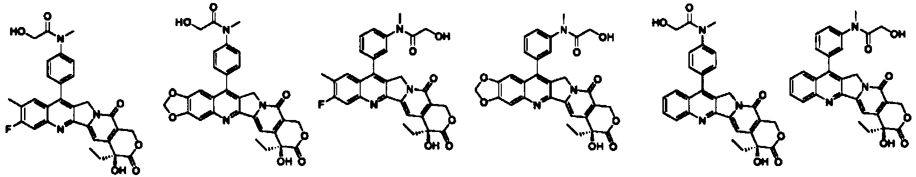

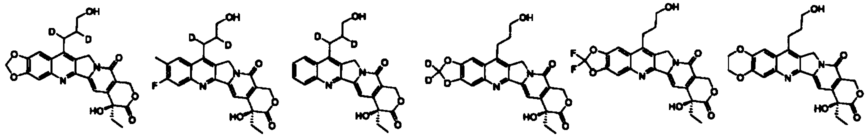

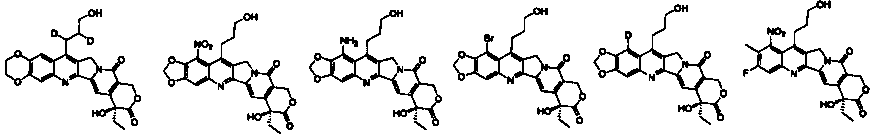

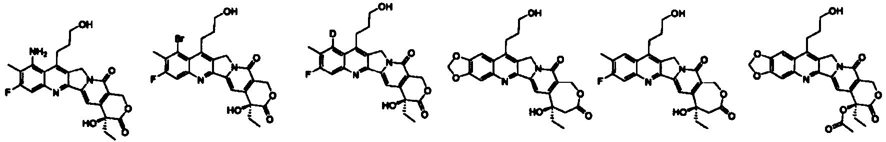

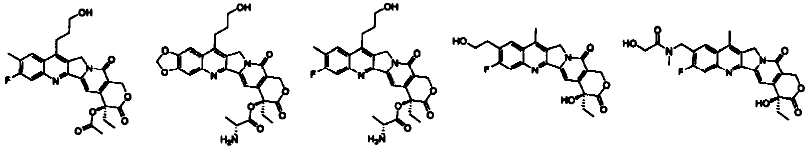

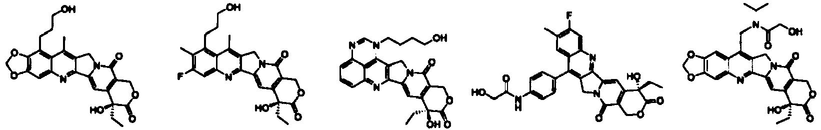

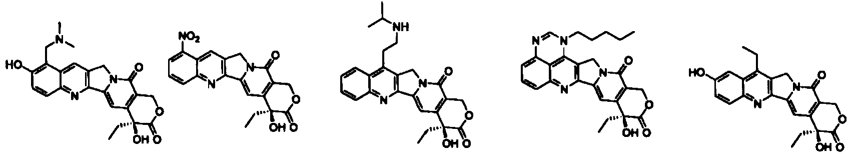

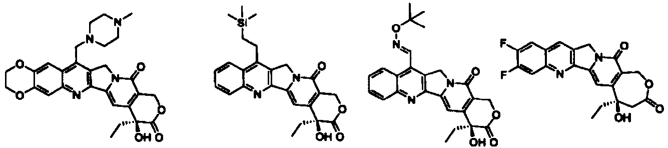

9. The pharmaceutical combination according to any one of claims 1-8, wherein the antibody-drug conjugate is selected from: 9MW-2921, A-315, ACR-246, ADC-2154, AZD-9592, AZD-9829, BIO-201, BLB-01D1, BSI-04702, CUSP-06, DAN-311, DB-1303, DB-1311, GPCR-targeted Project 010, HDP-201, HLX-42, HLX-43, IBI-354, IM-1021, JSKN-033, M-9140, MABS-01, MBK-101, MBK-102, MBK-105, NV-104, OBI-902, OBI-904, OBI-905, PRO-1102, DS-6000, IMMU-132, SMP-190, TQB-2102, DS-8201, DS-1062, DS-7300, U3-1402, XB-033, YL-201, YL-202, ZW-191, ZW-220, or ZW-251.

10. The pharmaceutical combination according to any one of claims 1-9, wherein the hyaluronidase is a mammalian hyaluronidase.

11. The pharmaceutical combination according to any one of claims 1-10, wherein the hyaluronidase comprises a catalytic domain of hyaluronidases PH-20, HYAL1, HYAL2, HYAL3, HYAL4, or HYALPS1.

12. The pharmaceutical combination according to any one of claims 1-11, wherein the hyaluronidase is selected from HuPH20, HYAL1, HYAL2, HYAL3, or HYAL4, or any variant thereof, or any isotype thereof.

13. The pharmaceutical combination according to any one of claims 1-12, wherein the hyaluronidase is a hyaluronidase set forth in any one of SEQ ID NOs: 1-3 or a fragment thereof.

14. The pharmaceutical combination according to any one of claims 1-13, wherein the pharmaceutical combination is selected from the group consisting of: rHuPH20 and DS-8201, rHuPH20 and IMMU-132, rHuPH20 and DS-1062, rHuPH20 and DS-7300, rHuPH20 and U3-1402, and PH20 variant 1 and DS-8201.

15. The pharmaceutical combination according to any one of claims 1-14, wherein the antibody-drug conjugate is administered at a concentration of about 2 - 100 mg/mL; and the hyaluronidase is administered at a concentration of about 100 - 5000 IU/mL;
optionally, the antibody-drug conjugate is administered at a concentration of about 2 mg/mL, 6 mg/mL, 8 mg/mL, 10 mg/mL, 20 mg/mL, 30 mg/mL, 40 mg/mL, 50 mg/mL, 60 mg/mL, 80 mg/mL, or 100 mg/mL; the hyaluronidase is administered at a concentration of about 100 IU/mL, 150 IU/mL, 200 IU/mL, 500 IU/mL, 1000 IU/mL, 2000 IU/mL, 3000 IU/ml, or 4000 IU/mL;
optionally, the antibody-drug conjugate is administered at a concentration of about 20 mg/mL, and the hyaluronidase is administered at a concentration of about 500 IU/mL.

16. The pharmaceutical combination according to any one of claims 1-14, wherein the antibody-drug conjugate and the hyaluronidase are administered in a dose ratio of about 2 - 100 mg : 100 - 5000 IU;
optionally, the antibody-drug conjugate and the hyaluronidase are administered in a dose ratio of about 3 - 60 mg : 100-3000 IU;
optionally, the antibody-drug conjugate is administered at a dose of 3 mg/mL, 4 mg/mL, 5 mg/mL, 6 mg/mL, 10 mg/mL, 20 mg/mL, 30 mg/mL, 40 mg/mL, 50 mg/mL, or 60 mg/mL;
optionally, the hyaluronidase is administered at a dose of 100 IU/mL, 150 IU/mL, 200 IU/mL, 500 IU/mL, 1000 IU/mL, 2000 IU/mL, or 3000 IU/mL;
optionally, a ratio of the antibody-drug conjugate to the hyaluronidase is about 20 mg : 500 IU.

17. The pharmaceutical combination according to any one of claims 1-16, wherein the antibody-drug conjugate is DS-8201.

18. The pharmaceutical combination according to any one of claims 1-17, wherein the pharmaceutical combination is: rHuPH20 and DS-8201; wherein DS-8201 is administered at a concentration of about 20 mg/mL, and rHuPH20 is administered at a concentration of about 500 IU/mL.

19. The pharmaceutical combination according to any one of claims 1-18, wherein the pharmaceutical combination is: rHuPH20 and DS-8201; wherein DS-8201 and rHuPH20 are administered in a dose ratio of about 20 mg : 500 IU.

20. A pharmaceutical composition for subcutaneous administration, comprising: the pharmaceutical combination according to any one of claims 1-19 and a pharmaceutically acceptable excipient.

21. A method for treating a tumor, comprising: administering to a subject in need the pharmaceutical combination according to any one of claims 1-19 or the pharmaceutical composition according to claim 20.

22. The method according to claim 21, wherein the tumor is lymphatic metastasis, breast ductal carcinoma, breast cancer, gastric cancer, ovarian cancer, or pancreatic cancer.

23. Use of the pharmaceutical combination according to any one of claims 1-19 or the pharmaceutical composition according to claim 20 in preparing a medicament for treating a tumor.
